# EUROPÄISCHE PATENTSCHRIFT

(11) **EP 1 130 026 B1**
(45) Veröffentlichungstag und Bekanntmachung des Hinweises auf die Patenterteilung: **22.09.2004**
(21) Anmeldenummer: 01103939.3
(22) Anmeldetag: 19.02.2001
(51) Int. Cl.: C07H 15/04, C11D 3/22

(54) **Gemische eines Ethylhexylglukosids mit hohem Oligomerisierungsgrad und konzentrierten Alkalilaugen**
Composition comprising ethylhexylglucosid with a high degree of oligomerization and concentrated alkali-hydroxides
Composition comprenant l'éthylhexylglucoside à degré d'oligomérisation élevé et hydroxides alcalins concentrés

(30) Priorität: 03.03.2000 DE 10010420
(43) Veröffentlichungstag der Anmeldung: 05.09.2001
(73) Patentinhaber: Goldschmidt AG, 45127 Essen (DE)
(72) Erfinder: Berkels, Wolfgang, 46238 Bottrop (DE); Grüning, Burghard, Dr., 45134 Essen (DE); Müller, Felix, Dr., 42555 Velbert (DE); Peggau, Jörg, 45357 Essen (DE)

(56) Entgegenhaltungen:
- EP-A- 0 729 970
- WO-A-93/07249
- WO-A-99/21948
- DE-A- 19 607 753
- US-A- 5 510 482
- US-A- 5 962 399

## Beschreibung

Gegenstand der Erfindung sind Gemische eines definierten Alkylpolyglucosids mit konzentrierten Alkalilauge, sowie die Verwendung dieser Gemische als Reinigungskonzentrat.

Die Reinigung im CIP-Verfahren (cleaning in place) erfolgt üblicherweise durch den Einsatz hochkonzentrierter technischer alkalischer Laugen, beispielsweise 50 Gew.-%ige Natronlauge oder 45 Gew.-%ige Kalilauge. In dem entsprechenden Betrieb, beispielsweise einer Brauerei oder Molkerei wird dieses Reinigungskonzentrat für die entsprechende Anwendung auf übliche Gebrauchskonzentrationen verdünnt. Zur Erhöhung der Reinigungsleistung werden üblicherweise weitere Tenside eingesetzt.

In der EP-A-0 729 970 wird ein Verfahren zur Herstellung von Alkylpolyglucosiden durch Umsetzung von Dextrose mit einem Überschuss von Fettalkoholen in Gegenwart von Säurekatalysatoren beschrieben.

Die US-A-5 510 482 beschreibt ein Bleichverfahren zur Behandlung der nach den Herstellungsverfahren anfallenden verfärbten rohen Alkylglucuoside.

Aus der WO 99/21948 ist es bekannt, dass Alkylpolyglucoside mit Hexylsubstitution in konzentrierten Alkalilaugen stabil sind. So wird auf S. 8 in der Tabelle ein N-Hexylglucosid beschrieben, das auch in 40 Gew.-%iger Natronlauge bei einer Konzentration von 7,5 % eine klare Lösung ergab. Angaben über den Oligomerisierungsgrad (DP) sind in dieser Schrift nicht enthalten.

Das in United States Statutory Invention Registration H171 beschriebene Produkt ist ebenso nicht geeignet für die Kombination mit hochkonzentrierten Alkalilaugen.

In der DE-A-196 07 753 werden Alkylpolyglucoside mit einem hohen Oligomerisierungsgrad als leistungsstarke Tenside für flüssige Spül- und Reinigungsmittel beschrieben. Es wird hier beschrieben, dass entsprechende Alkylpolyglucoside dadurch zugänglich sind, dass man Fettalkohole und Glucose im molaren Einsatzverhältnis 3 : 1 bis 10 : 1 bei Temperaturen im Bereich von 90 bis 120 °C in einer sauer katalysierten Acetalisierung unter kontinuierlicher Abdestillation des Reaktionswassers zu Alkyloligoglucosiden mit niedrigem Oligomerisierungsgrad (< 1,6) verarbeitet und nach Abschluss der Reaktion den sauren Katalysator zu 0 bis 90 mol.-% neutralisiert.

Nach der Abtrennung einer solchen Menge nicht umgesetzten Fettalkohols, die das molare Verhältnis des noch verbliebenen Fettalkohols und der Glucose, bezogen auf die Ausgangsmenge, auf 1 : 1 bis 3 : 1 reguliert, wird die Reaktionsmischung einer Nachpolymerisation bei Temperaturen im Bereich von 90 bis 120 °C unterworfen.

Nach der Neutralisation der verbleibenden Menge des sauren Katalysators und Abtrennung des nicht umgesetzten Fettalkohols erhält man Alkyloligoglucoside, die einen Oligomerisierungsgrad im Bereich von 1,6 bis 2,5, insbesondere 1,6 bis 2 aufweisen, ohne dass die Produkte durch einen hohen Polyglucosegehalt oder eine unzureichende Farbqualität belastet wären. Dabei konnte der Anteil der Polyglucose auf unter 5 % gesenkt werden.

Typische Beispiele für Fettalkohole, die bei diesem Verfahren als Ausgangsstoff eingesetzt werden können, sind Capronalkohol, Caprylalkohol, 2-Ethylhexylalkohol, Caprinalkohol, Laurylalkohol, Isotridecylalkohol, Myristylalkohol, Cetylalkohol, Stearylalkohol, Isostearylalkohol und Behenylalkohol sowie deren Mischungen, die beispielsweise bei der Hochdruckhydrierung von technischen Methylestern auf der Basis von Fetten und Ölen oder Aldehyden aus der Roelen'schen Oxosynthese sowie als Monomerfraktion bei der Dimerisierung von ungesättigten Fettalkoholen anfallen.

Als besonders bevorzugte technische Fettalkohole werden solche mit 8 bis 10 bzw. 12 bis 18 C-Atomen, insbesondere gehärteter Vorlauf-, Kokos-, Palm-, Palmkern- oder Talgfettalkohol eingesetzt.

Als saure Katalysatoren werden insbesondere Schwefelsäure, Alkylschwefelsäurehalbester, Alkylbenzolsulfonsäure, wie beispielsweise p-Toluolsulfonsäure oder Dodecylbenzolsulfonsäure und Sulfobernsteinsäure eingesetzt. Die Einsatzmengen liegen üblicherweise im Bereich von 0,1 bis 5, vorzugsweise 0,5 bis 2 Gew.-%, bezogen auf die Einsatzstoffe.

Die in dieser Schrift konkret offenbarten Tenside sind jedoch nicht geeignet für hochkonzentrierte alkalische Reinigerkonzentrate.

Dementsprechend besteht ein Bedürfnis, ein hochstabiles Polyalkylglucosid zur Verfügung zu stellen, das in handelsüblichen konzentrierten technischen Alkalilaugen beständig ist und gleichzeitig eine gute Emulgierleistung aufweist.

Die vorgenannte Aufgabe wird erfindungsgemäß gelöst durch ein Alkylpolyglucosid mit einem Oligomerisierungsgrad (DP) von wenigstens 1,7 bis 3, wobei der Alkylrest 8 C-Atome umfasst. Erfindungsgemäß wird somit Ethylhexylglucosid mit einem Oligomerisierungsgrad im Bereich von 1,7 bis 3 beschrieben.

Überraschenderweise wurde gefunden, dass dieses Alkylpolyglucosid auch in hochkonzentrierten Alkalilaugen auf der Basis von Natronlauge oder Kalilauge langzeitstabil ist und hier zu keinen Ausfällungen führt. Auch die Emulgierleistung ist in verdünnten alkalischen Reinigungslösungen gut.

Das erfindungsgemäße Alkylpolyglucosid ist nach einem Verfahren erhältlich, das in der DE-A-196 07 753 im Detail beschrieben wird. Insoweit wird voll inhaltlich Bezug auf diese Schrift genommen.

In einer bevorzugten Ausführungsform der vorliegenden Erfindung weist das erfindungsgemäße Alkylpolyglucosid einen Oligomerisierungsgrad (DP) von 1,85 bis 2,5 auf.

Im Stand der Technik ist es bekannt, Reinigungskonzentrate auf der Basis von Alkylpolyglucosiden mit Alkylresten einzusetzen, bei denen die Anzahl der C-Atome im Alkylrest von 8 verschieden ist (siehe beispielsweise DE-A-196 07 753). So sind aus dem genannten Stand der Technik Alkylpolyglucoside mit einem Alkylrest, der insbesondere 6 bis 24 C-Atome umfasst, bekannt. Diese an sich bekannten Alkylpolyglucoside können im Sinne der vorliegenden Erfindung in Kombination mit dem Ethylhexylglucosid eingesetzt werden. Besonders bevorzugt im Sinne der vorliegenden Erfindung sind daher Gemische von Alkylpolyglucosiden anderer Provenienz mit dem Ethylhexylglucosid, wobei das Gewichtsverhältnis des Ethylhexylglucosids zu den weiteren Alkylpolyglucosiden so eingestellt wird, dass wenigstens 50 Gew.-% des Alkylpolyglucosids aus Ethylhexylglucosid bestehen.

Zum Einsatz des erfindungsgemäßen Alkylpolyglucosids und der Gemische dieses Alkylpolyglucosids mit weiteren Alkylpolyglucosiden, werden diese mit konzentrierter technischer Alkalilauge versetzt. Handelsübliche technische konzentrierte Alkalilaugen umfassen beispielsweise 50 Gew.-% NaOH oder auch 45 Gew.-% KOH in Wasser.

Besonders bevorzugt im Sinne der vorliegenden Erfindung sind daher Gemische aus einem oder mehreren der genannten Alkylpolyglucoside mit technischen konzentrierten Alkalilaugen, wobei diese 50 bis 99,9 Gew.-% konzentrierte Alkalilaugen und 0,1 bis 50 Gew.-% der Alkylpolyglucoside enthalten. Besonders bevorzugt im Sinne der vorliegenden Erfindung sind entsprechende Gemische, die 90 bis 99,9 Gew.-% technische konzentrierte Alkalilaugen und 0,1 bis 10 Gew.-% der Alkylpolyglucoside enthalten.

Besonders bevorzugt im Sinne der vorliegenden Erfindung werden die vorgenannten Reinigungsmittel-Konzentrate zur Reinigung von Oberflächen in Brauereien und Molkereien eingesetzt.

Das erfindungsgemäße Alkylpolyglucosid ist deutlich schaumärmer, jedoch wird die Oberflächenaktivität nicht negativ beeinflusst.

### Ausführungsbeispiele:

### Beispiel 1/Vergleichsbeispiele 1 bis 7:

Analog der DE 196 07 753 A1 wurden die in der nachfolgenden Tabelle 1 genannten Alkylpolyglucoside (APG) hergestellt:

**Tabelle 1:**

| Beispiele: | Verbindung: | Oligomerisierungsgrad: |
|---|---|---|
| Beispiel 1 | EthylhexylPG | ca. 2,0 |
| Vergleichsbeispiel 1 | EthylhexylPG | ca. 1,1 |
| Vergleichsbeispiel 2 | EthylhexylPG | ca. 1,3 |
| Vergleichsbeispiel 3 | EthylhexylPG | ca. 1,6 |
| Vergleichsbeispiel 4 | Ethylhexyl-/Decyl-PG | ca. 1,5 |
| Vergleichsbeispiel 5 | Octyl-Decyl-PG | ca. 1,6 |
| Vergleichsbeispiel 6 | Octyl-Decyl-PG | ca. 1,6 |
| Vergleichsbeispiel 7 | Octyl-Decyl-PG* | ca. 1,6 |

| | | |
|---|---|---|
| * Gemäß US 1985-801170 | | |

In der nachfolgenden Tabelle 2 wird das Löslichkeitsverhalten der oben beschriebenen Alkylpolyglucoside in konzentrierter wässriger Natronlauge untersucht:

**Tabelle 2:**

| Beispiele: | Gelöst in Gew.-% NaOH | | | | | | |
|---|---|---|---|---|---|---|---|
| | 15 | 20 | 25 | 30 | 35 | 40 | 50 |
| Beispiel 1 | + | + | + | + | + | + | + |
| Vergleichsbeispiel 1 | - | - | - | - | - | - | - |
| Vergleichsbeispiel 2 | + | + | - | - | - | - | - |
| Vergleichsbeispiel 3 | + | + | + | - | - | - | - |
| Vergleichsbeispiel 4 | + | + | + | + | + | ± | - |
| Vergleichsbeispiel 5 | + | + | ± | - | - | - | - |
| Vergleichsbeispiel 6 | + | + | + | - | - | - | - |
| Vergleichsbeispiel 7 | + | + | + | - | - | - | - |

In gleicher Weise wurde auch die Löslichkeit in konzentrierter Kalilauge bestimmt. Die Ergebnisse sind hier in der Tabelle 3 wiedergegeben:

**Tabelle 3:**

| Beispiele: | Gelöst in Gew.-% KOH | | | | | | |
|---|---|---|---|---|---|---|---|
| | 15 | 20 | 25 | 30 | 35 | 40 | 45 |
| Beispiel 1 | + | + | + | + | + | + | + |
| Vergleichsbeispiel 4 | + | + | + | + | + | + | ± |

In einer weiteren Versuchsreihe wurde die Emulgierfähigkeit der obengenannten Alkylpolyglucoside in einer Verdünnung von 1,0 g/l in Olivenöl bestimmt:

Die Emulgierleistung wurde bestimmt durch Herstellung einer 1:1 Mischung von Wasser und handelsüblichem Olivenöl. Hierzu gab man 0,05 % der Alkylpolyglucoside und mischte anschließend unter intensivem Durchschütteln. Die Abscheidung von Öl wurde in verschiedenen Zeitschritten beobachtet. Je geringer die Ölabscheidung war, desto leistungsfähiger war das Tensid.

Mit den Alkylpolyglucosiden der Vergleichsbeispiele 1 bis 3 und 5 bis 7 konnten keine Emulsionen erhalten werden.

Die nachfolgende Tabelle 4 gibt die erhaltenen Daten wieder:

**Tabelle 4:**

| Beispiele | % Öl-Separation | | | |
|---|---|---|---|---|
| | 30 min | 1 h | 2 h | 3 h |
| Beispiel 1 | 2 | 4 | 8 | 12 |
| Vergleichsbeispiel 4 | 1 | 6 | 10 | 20 |

In der nachfolgenden Tabelle sind Formulierungen der alkalischen Reiniger mit maximaler NaOH-Konzentration wiedergegeben:

**Tabelle 5:**

| Beispiele | NaOH 50%ig | Gluconsäure 50%ig | Menge des APG^{*} | Wasser |
|---|---|---|---|---|
| Beispiel 1* | 80 | 10,0 | 5,0 | - |
| Vergleichsbeispiel 4* | 70 | 10,0 | 5,0 | auf 100 |
| Vergleichsbeispiel 6 | 60 | 10,0 | 5,0 | auf 100 |
| Vergleichsbeispiel 7 | 60 | 10,0 | 5,0 | auf 100 |

| | | | | |
|---|---|---|---|---|
| * auf 100 % Wirksubstanz | | | | |

In der nachfolgenden Tabelle ist die Emulgierfähigkeit alkalischer Reiniger in Olivenöl in einer 1%igen Verdünnung [enthält 0,5g/l des APG in der getesteten Lösung] angegeben:

**Tabelle 6:**

| Beispiele | % Wasser-Separation | | | |
|---|---|---|---|---|
| | 30 min | 1 h | 3 h | 24 h |
| Beispiel 1 | 0 | 0 | 8 | 40 |
| Vergleichsbeispiel 4 | 0 | 0 | 12 | 54 |
| Vergleichsbeispiel 6 | 0 | 0 | 10 | 50 |
| Vergleichsbeispiel 7 | 0 | 0 | 14 | 56 |

In der nachfolgenden Tabelle ist die Emulgierfähigkeit alkalischer Reiniger in Olivenöl in einer 0,5%igen Verdünnung [enthält 0,25g/l des APG in der getesteten Lösung] angegeben:

**Tabelle 7:**

| Beispiele | % Wasser-Separation | | | |
|---|---|---|---|---|
| | 30 min | 1 h | 3 h | 24 h |
| Beispiel 1 | 0 | 1 | 8 | 40 |
| Vergleichsbeispiel 4 | 0 | 2 | 8 | 40 |

In der nachfolgenden Tabelle ist die Emulgierfähigkeit alkalischer Reiniger in Olivenöl in einer 0,25%igen Verdünnung [enthält 0,125g/l des APG in der getesteten Lösung] angegeben:

**Tabelle 8:**

| Beispiele | % Wasser-Separation | | | |
|---|---|---|---|---|
| | 30 min | 1 h | 3 h | 24 h |
| Beispiel 1 | 0 | 1 | 6 | 30 |
| Vergleichsbeispiel 4 | 0 | 2 | 6 | 30 |

In der nachfolgenden Tabelle ist die Emulgierfähigkeit alkalischer Reiniger in Olivenöl in einer 0,1%igen Verdünnung [enthält 0,05g/l des APG in der getesteten Lösung] angegeben:

**Tabelle 9:**

| Beispiele | % Wasser-Separation | | | |
|---|---|---|---|---|
| | 30 min | 1 h | 3 h | 24 h |
| Beispiel 1 | 0 | 2 | 6 | 0 |
| Vergleichsbeispiel 4 | 0 | 2 | 6 | 4ml Öl |

In der nachfolgenden Tabelle ist das Schaumverhalten (DIN 53902) von 1,0g/l EthylhexylPG verschiedener DP-Grade angegeben:

Das Schaumvermögen wurde in Anlehnung an DIN 53902 Teil 1 bestimmt, es handelte sich hierbei um das Lochscheiben-Schlagverfahren in der Hand-Schlagmethode.

In einen Standzylinder (Größe 500 ml) mit cm-Einteilung wurde 250 ml Tensidlösung gefüllt, dies entsprach einem Flüssigkeitsbestand von 14 cm. Der Lochstempel (Scheibendurchmesser 45 mm mit 12 Bohrungen je 5 mm) wurde auf den Zylinder gesetzt und gleichmäßig 20 Schläge ausgeführt. Der hierdurch erzeugte Schaum wurde in cm-Schaumhöhe angegeben. Wobei der Wert direkt nach dem Aufschlagen als Gesamthöhe (Flüssigkeit und Schaum) und bei den Werten für 30, 60 und 120 Sekunden nur die eigentliche Schaumhöhe angegeben wurde.

**Tabelle 10:**

| Beispiele | Schaumhöhe [cm] | | | |
|---|---|---|---|---|
| | Anfang | 30" | 60" | 120'' |
| Beispiel 1 | 10 | 1,5 | 1 | 1 |
| Vergleichsbeispiel 1 | 14 | 2 | 1 | 1 |
| Vergleichsbeispiel 2 | 13 | 2 | 1 | 1 |
| Vergleichsbeispiel 3 | 12 | 1,5 | 1 | 1 |

In der nachfolgenden Tabelle ist das Schaumverhalten (DIN 53902) alkalischer Reiniger mit dem APG wiedergegeben:

**Tabelle 11:**

| Beispiele | Konz. | Schaumhöhe [cm] | | | |
|---|---|---|---|---|---|
| | | Anfang | 30'' | 60'' | 120'' |
| Beispiel 1* | 0,50% | 10 | 2 | 1 | 1 |
| Vergleichsbeispiel 4* | 0,50% | 13 | 11 | 10 | 10 |
| Beispiel 1* | 0,25% | 8 | 1 | 0,5 | 0,5 |
| Vergleichsbeispiel 4* | 0,25% | 9 | 7 | 6 | 6 |
| Beispiel 1* | 0,10% | 5 | 0 | 0 | 0 |
| Vergleichsbeispiel 4* | 0,10% | 7 | 5 | 4 | 3,5 |

| | | | | | |
|---|---|---|---|---|---|
| * Betrifft die Formulierungen gemäß Tabelle 5 | | | | | |

In der nachfolgenden Tabelle wird die Oberflächenspannung von 1,0 g/l EthylhexylPG verschiedener DP-Grade angegeben:

Verwendet wurde das Digitaltensiometer K10ST der Firma Krüss. Als Messkörper wurde ein Ring, nach Du Nouy, verwendet.

Der Platinring wurde in die zu untersuchende Flüssigkeit eingetaucht und anschließend wieder herausgezogen. Gemessen wurde die Kraft, die erforderlich war, um den Ring durch die Oberfläche zu ziehen.

**Tabelle 12:**

| Beispiele | Oberflächenspannung [mN/cm] |
|---|---|
| Beispiel 1 | 33 |
| Vergleichsbeispiel 1 | 34 |
| Vergleichsbeispiel 2 | 33 |
| Vergleichsbeispiel 3 | 32 |

In der nachfolgenden Tabelle wird die Oberflächenspannung alkalischer Reiniger mit dem APG wiedergegeben:

**Tabelle 13:**

| Beispiele | Konz. | Wirkstoff-Gehalt APG | Oberflächenspannung [mN/cm] |
|---|---|---|---|
| Beispiel 1* | 0,50% | 0,25g/l | 35 |
| Vergleichsbeispiel 4* | 0,50% | 0,25g/l | 28 |
| Beispiel 1* | 0,25% | 0,125g/l | 43 |
| Vergleichsbeispiel 4* | 0,25% | 0,125g/l | 30 |
| Beispiel 1* | 0,10% | 0,05g/l | 47 |
| Vergleichsbeispiel 4* | 0,10% | 0,05g/l | 34 |

| | | | |
|---|---|---|---|
| * Betrifft Formulierungen gemäß Tabelle 5 | | | |

## Patentansprüche

1. Gemische eines oder mehrerer Ethylhexylglucoside mit einem Oligomerisierungsgrad (DP) von wenigstens 1,7 bis 3 und konzentrierten Alkalilaugen.

2. Gemische nach Anspruch 1, **dadurch gekennzeichnet, dass** sie 50 bis 99,9 Gew.-% konzentrierte Alkalilaugen und 0,1 bis 50 Gew.-% Ethylhexylglucosid enthalten.

3. Gemische nach Anspruch 1, **dadurch gekennzeichnet, dass** sie 90 bis 99,9 Gew.-% konzentrierte Alkalilaugen und 0,1 bis 10 Gew.-% Ethylhexylglucosid enthalten.

4. Verwendung nach einem der Ansprüche 1, 2 oder 3 als Reinigungskonzentrat für die industrielle Reinigung von Oberflächen.

## Claims

1. Mixtures of one or more ethylhexylglucosides with a degree of oligomerization (DP) of at least 1.7 to 3 and concentrated alkali metal hydroxide solutions.

2. Mixtures according to Claim 1, **characterized in that** they comprise 50 to 99.9% by weight of concentrated alkali metal hydroxide solutions and 0.1 to 50% by weight of ethylhexylglucoside.

3. Mixtures according to Claim 1, **characterized in that** they comprise 90 to 99.9% by weight of concentrated alkali metal hydroxide solutions and 0.1 to 10% by weight of ethylhexylglucoside.

4. Use according to one of Claims 1, 2 or 3 as cleaner concentrate for the industrial cleaning of surfaces.

## Revendications

1. Mélanges d'un ou de plusieurs éthylhexylglucosides présentant un degré d'oligomérisation (DP) d'au moins 1,7 à 3 et de lessives alcalines concentrées.

2. Mélanges selon la revendication 1, **caractérisés en ce qu'**ils contiennent 50 à 99,9% en poids de lessives alcalines concentrées et 0,1 à 50% en poids d'éthylhexylglucoside.

3. Mélanges selon la revendication 1, **caractérisés en ce qu'**ils contiennent 90 à 99,9% en poids de lessives alcalines concentrées et 0,1 à 10% en poids d'éthylhexylglucoside.

4. Utilisation selon l'une quelconque des revendications 1, 2 ou 3 comme concentrat de nettoyage pour le nettoyage industriel de surfaces.
